(19) Europäisches Patentamt
European Patent Office
Office européen des brevets

(11) Publication number : **0 301 068 B1**

(12) **EUROPEAN PATENT SPECIFICATION**

(45) Date of publication of patent specification : **12.08.92 Bulletin 92/33**

(51) Int. Cl.$^5$ : **G01N 1/28,** G01N 33/04

(21) Application number : **88901810.7**

(22) Date of filing : **08.02.88**

(86) International application number : **PCT/DK88/00015**

(87) International publication number : **WO 88/05906 11.08.88 Gazette 88/18**

# (54) A METHOD AND AN APPARATUS FOR HEATING LIQUID SAMPLES, AND A CONTAINER FOR USE IN THE APPARATUS.

(30) Priority : **06.02.87 DK 605/87**

(43) Date of publication of application : **01.02.89 Bulletin 89/05**

(45) Publication of the grant of the patent : **12.08.92 Bulletin 92/33**

(84) Designated Contracting States : **AT BE CH DE FR GB IT LI LU NL SE**

(56) References cited :
**EP-A- 0 156 742**
**EP-A-01 364 53**

(56) References cited :
**WO-A-60/1065**
**DE-A- 3 325 195**
**DE-B-28 273 26**
**DK-B-11 367 7**

(73) Proprietor : **Hanséns Verkstäder AB**
**Hedentorpsv. 8**
**S-291 21 Kristianstad (SE)**

(72) Inventor : **Petersen, Olav**
**Slotsherrensvej 4**
**DK-3400 Hillerod (DK)**

(74) Representative : **WITTRUP, Flemming et al**
**c/o Hofman-Bang & Boutard A/S Adelgade 15**
**DK-1304 Copenhagen K (DK)**

EP 0 301 068 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The invention concerns a method of heating liquid samples contained in containers to a predetermined temperature, wherein the containers are introduced into a heating chamber. The method is particularly useful for heating milk to be subjected to treatment in an analyzer, where a large numer of samples, which may have different initial temperatures and liquid amounts, are to be analysed, it being presupposed that when taken for analysis all samples have the same predetermined temperature.

In respect of milk samples, the samples to be heated are generally taken with at least one sample per milked cow and are sent, together with the milk supply, to the dairy where they are analysed. The result of the analysis forms the basis for the settlement with the supplier. Thus, it is a large number of

same heating chamber. In this system it is not possible to sense the individual temperatures and therefore no individual heating can take place.

The object of the invention is to provide a method of the stated type which, while requiring little space, makes it possible to heat a large number of samples per unit of time exactly to a predetermined temperature, and also enables a construction of the apparatus necessary for performing the method which is acceptable to the user and the environments. This is achieved in that the method is performed as stated in claim 1, the high frequency energy causing rapid and uniform heating of the whole liquid sample, and this energy impact is discontinued when the container leaves the impact zone. The stated method also eliminates the drawback of the conventional heating method that the containers have to stay in water, which has an adverse effect on their identification

Claim 9 defines a simple and effective embodiment of the holding means for maintaining the platforms in the lifted position and for releasing them from this position to remove the containers from the heating chamber under the action of gravity when the reference temperature has been reached.

The invention further concerns a container for use in the stated apparatus and consisting of a material of poor heat conduction, and when this container is constructed as stated in the characterizing portion of claim 10, good heat transfer contact is obtained between the temperature sensor of the apparatus and the sample liquid in the container irrespective of the poor heat conductivity of the container material. Seen from a production-technical point of view, it is usually most expedient that the metal disc is arranged in the container cover. The fact that the containers will then have to be arranged bottom up on the platforms involves the additional advantage that the reversal of the container provides a certain stirring effect in and thus greater uniformity of the sample liquid

The invention will be explained more fully below with reference to the drawing, in which

fig. 1 is a developed vertical section through part of the embodiment of the apparatus of the invention,

fig. 2 is a vertical section through the entire apparatus,

fig. 3 is a top view of the transport mechanism of the apparatus, and

fig. 4 is a vertical section through one of the lifting mechanisms of the apparatus.

In the drawing, 1 is a stationary machine frame, in relation to which an assembly 2, which constitutes the major part of the apparatus, can move in the direction indicated by an arrow. As shown in figs. 2 and 3, the machine frame 1 and the assembly 2 may be constructed as a circular unit, the assembly 2 being rotatable about a bearing in the machine frame. This arrangement represents a preferred embodiment, and the apparatus will be described in this form below. The centre of the machine frame incorporates a central pipe 16, fig. 2, carrying a bearing 17 in which the assembly 2 is rotatably journalled. The assembly 2 is provided with a toothed rim 15 which meshes with a gear wheel 14 arranged on the shaft of an engine 12. The engine 12 is firmly positioned in connection with the machine frame 1. When the engine is activated, the entire assembly 2 consequently rotates with respect to the machine frame about the bearing 17. The shaft of the engine 12 also carries a transport wheel 13 so constructed as to be capable of transporting sample containers 11 containing the sample to be heated from a feed belt 18 into the assembly 2. The transport wheel 13 is coupled to the assembly 2 such that an introduced sample container will be delivered on one of a plurality of platforms having lifting means, whose arrangement will be explained more fully later. If the feed belt 18 is full, the assembly 2 will consequently be filled with sample containers when the engine 12 is activated. The toothed rim 15 is moreover coupled with a transport wheel 20 adapted to transport the sample containers from the assembly 2 out on to a discharge belt 19 which can transport the sample containers away. This transport wheel is arranged so that the sample containers traverse such a great part of an entire rotation of the assembly 2 as is geometrically possible.

As shown in fig. 2, the assembly 2 is divided into four "floors" or levels I-IV. Level I is the top one and is called weight chamber level for reasons given below. Level II is called heating chamber level below. It constitutes the part in which the material to be heated is placed and contains a high frequency apparatus which can deposit energy in materials having suitable dielectric properties.

In a preferred embodiment, the high frequency apparatus itself is formed by a microwave oven, but nothing prevents the use of a dielectric high frequency heater. In the first case, the heating chamber will be in the form of a suitably dimensioned space where the high frequency energy is fed from a wave conductor. In the second case, the heating chamber is formed as a co-axial capacitor which is incorporated in an oscillation circuit in the power generator.

Level III is called transport level below and is the level where the sample containers are present when they have just been introduced into the apparatus and when, after complexed heating, they are transported out of the apparatus. Level IV is called elevator level and contains the lifting means and guides which just allow axial movements of the lifting means, and means for performing this movement.

It is schematically shown in fig. 1 haw the sample containers can be transported through the apparatus. Sample containers 11 are shown in a plurality of different positions during the transport through the apparatus. To the extreme right, a container has just been delivered from the transport wheel and stands an its platform 29. This is shown in more detail in fig. 4, together with the associated lifting means 24 and other components. The lifting means 24 may be moved vertically by means of a ramp 6 on the machine frame and thus be lifted from the elevator level IV upwardly toward the heating chamber level II, so that the container is introduced into a heating chamber 7 when the assembly 2 is moved in the direction of the arrow. A holding mechanism, generally designated by 23, serves to retain the lifting .means in its top position and may e.g. be released by an electric magnet. The lifting means will be retained by the holding means 23 in a position where the sample container 11 is present in the heating chamber when it is no longer under the action of the ramp 6. The heating chamber receives power from the high frequency apparatus, and sample containers 11 present in the heating chamber will therefore be heated.

Co-axially with each sample position, a weight 9, guided be a weight guide 10, is present in level I. When no sample container is present in the sample position in question, the weight is in its lowermost position and thus closes the access to the heating chamber, resulting in a considerable reduction in unintentional radiation of high frequency energy to the surroundings. When a sample container is introduced into the heating chamber by its lifting means, the container displaces the weight so that this partly moves upwardly in its guide and partly subjects the container to the load of its weight, so that the container is maintained steadily and is brought into good heat-conduction contact with a temperature sensor 5 disposed at the top end of the lifting means. When the holding mechanism 23 is released, the weight finally contributes to rapid ejection of the container from the heating chamber and causes it to be closed again to the surroundings.

An electronic unit, fed with power via slip rings, is placed in level I. The electronic unit contains a circuit for each sample position, said circuit comparing a signal from each temperature sensor with an adjustable reference signal adjusted to a value which corresponds to the signal value of the temperature sensor for the desired temperature of the samples. The comparator circuit may be formed by an operational amplifier of a generally known type, which receives the reference signal on its one input and receives the signal from the temperature sensor on its other input. A suitable temperature sensor is e.g. a thermistor of a type which is used for disposable thermometers for medical purposes. Such a thermistor is accurately calibrated precisely in the temperature range which is relevant here, viz. 40 to 42°C, and such precise calibration is desirable, particularly if it is desired to use a common reference for all the comparator circuits. The comparator circuit has its output connected to an amplifier which controls the holding mechanism such that it maintains the sample in the heating chamber until the temperature sensor indicates that the sample has obtained the temperature prescribed by the reference. The holding mechanism 23 is so constructed in a preferred embodiment that it has to be fed with power to release the sample so that it is ejected from the heating chamber, and in this case a monostable multivibrator may be interposed between the comparator circuit and the amplifier, said multivibrator being triggered by the comparator circuit when the sample has reached the desired temperature so that the holding mechanism is just fed with power for a period of sufficient duration to ensure certain release of the sample.

Fig. 4 shows a preferred embodiment of the means to introduce the samples into the heating chamber and to maintain them as well as to release them when they have reached the desired temperature.

The arrangement is shown in the state it has when a sample has just been delivered from the transport wheel. As mentioned, the sample is delivered on a platform, which is ring-shaped and stands, in its position of rest, on a partition between the elevator level IV and the transport level III. The platform 29 is kept radially in place by the lifting means 24 which extends through the hole in the centre of the platform with a loose fit. Axially, the platform is retained with respect to the lifting means by a locking ring 30 which projects into a recess in the inner side of the platform, said recess being so shaped as to allow a certain axial movement. On the platform, the temperature sensor 5 is placed with the active sensor element in the centre of the centre hole of the platform and is so loosely suspended as to be disposed below the top side of the platform in its position of rest and thus allows unobstructed delivery of the sample on the platform. The temperature sensor is connected to the electronic unit by a lead 32. The lifting means 24 is guided by a guide in the partition 28 and by a guide column 33 so that while being readily axially movable it is precisely guided and controlled. The ramp 6 on the machine frame cooperates with the lifting means 24 as shown. When the assembly 2 is rotated with respect to the machine frame, the lifting means will thus be displaced vertically, so that, owing to the allowed axial movement between platform and lifting means, the temperature sensor will be pushed upwardly toward and be brought into good heat conducting contact with a metallic membrane provided on the cover of the sample container,and the container will additionally be moved up into the heating chamber against the load of the weight 9. A clamp plate 34, loosely suspended from a rod 35, allows movement of the lifting means in an upward direction, but opposes by clamping effect any movement of the lifting means in a downward direction and thus causes the sample to be retained in the heating chamber, also when the lifting means is no longer under the action of the ramp 6. The clamp plate 34 is also mechanically connected to a solenoid 36. When this solenoid is energized, it lifts the clamp plate and thus causes release of the clamping effect of the clamp plate, so that the sample is ejected from the heating chamber under the load of the weight 9. The fall of the sample toward the transport level is cushioned by the air around the column 33, and to provide suitable cushioning a suitable blow-off hole may optionally be formed in the lifting means.The solenoid is connected to the electronic unit and will be activated when the temperature sensor indicates that the sample has reached the temperature prescribed by the reference. It is important to ensure that all samples are present in the transport level when they are to be transported out of the apparatus, even under possible error conditions. A boss 37, is so placed on the machine frame as to cooperate with the clamp plate to forcibly cancel its clamping effect so late in the cycle that all samples should have been ejected after the normal function of the machine.In fig.4, the toothed rim 15 and the gear wheel 14 are shown disposed below the bottom of the assembly 2. When these components are positioned as shown, the transport wheels 13 and 20 are synchronized with the carrousel without additional gearing beteween gear wheel and transport wheel to obtain correct geometry.

The invention is not restricted to the special embodiment shown and described, since its details may be modified in many ways within the scope of the invention, just as the method and the apparatus of the invention may be used for heating other liquids than milk.

## Claims

1. A method for heating liquid samples in respective containers (11) to a predetermined reference temperature, comprising,

heating the liquid samples with high frequency energy in a heating chamber (7) common for a plurality of liquid samples,

introducing the liquid samples sequentially into the heating chamber via a plurality of sample handling means,

each one receiving one of said samples ;

each one sealing the heating chamber (7) with respect to high frequency energy each time one liquid sample has been received in the heating chamber;

each one sensing the temperature of the respective liquid sample during the heating and comparing the sensed temperature with a predetermined reference temperature;

each one removing its respective sample individually from the heating chamber in dependence on said comparison;

and each one sealing the heating chamber (7) with respect to high frequency energy each time each liquid sample has been removed from the heating chamber (7);

while liquid samples remaining in the heating chamber (7) are still heated.

2. A method according to claim 1, **characterized** by successively lifting the containers (11) up into the heating chamber (7) from a transport means (28) extending below and along the heating chamber, and releasing each container for return to the transport means by means of gravity when its contents have reached the reference temperature.

3. A method according to claim 2, **characterized** in that the heating chamber (7) and the transport means (28) move as a unit in a ring-shaped path.

4. An apparatus for heating liquid samples in respective containers (11) to a predetermined reference temperature and comprising a heating chamber (7) in which the liquid samples are heated with high frequency energy,

said heating chamber (7) comprising a plurality of sample positions for samples to be heated,

characterised in that the apparatus comprises a plurality of sample handling means for sequentially introducing the containers (11) into the heating chamber (7), each individual sample handling means comprising :

means for sealing the heating chamber (7) with respect to high frequency energy each time a sample has been received in the heating chamber (7);

a sensor (5) for sensing the temperature of each individual liquid sample during the heating;

a mechanism for discharging the respective containers (11) from the heating chamber (7) when the liquid sample has reached its reference temperature;

and sealing means for high frequency energy sealing said heating chamber, said sealing means being operatively connected with ist respective discharging mechanism for sealing the heating chamber when the container (11) has been removed from the heating chamber (7),

so that the heating of the remaining samples can be continued.

5. An apparatus according to claim 4, **characterized** in that it has a ring-shaped transport means (28) rotatable about a vertical axis and formed with spaced, vertically movable platforms (29) to support their respective containers (11), and that the heating chamber (7) is firmly connected with the transport means and disposed at a distance greater than the container height above the platforms when these are in their bottom positions, means (6, 24) being provided to lift each platform (29) such that a container (11) supported thereby is moved up into the heating chamber (7) through an opening in its bottom, releasable holding means (34) being provided to retain each container (11) in the heating chamber (7) until the contained liquid sample has reached the reference temperature.

6. An apparatus according to claim 5, **characterized** in that a temperature sensor (5) is so placed on each platform (29) as to contact a container (11) placed on the platform (29).

7. An apparatus according to claim 6, **characterized** in that each platform (29) is constituted by a carrier ring and a rod-shaped, axially movable lifting means (24) whose upper end part extends up into and is so connected with the ring that the parts have limited axial movablility in relation to each other, said temperature sensor (5) being so placed on the end of the lifting means as to be present below and above the support face of the carrier ring, respectively, when the lifting rod is in its bottom position and top position, respectively, with respect to the carrier ring, a stationary ramp (6) being provided to cause upward movement of the rod-shaped lifting means.

8. An apparatus according to claim 5, 6, or 7, **characterized** in that the heating chamber (7) is upwardly defined by a ceiling plate having openings coaxial with the openings in the bottom, and that a weight (9) is axially movable in each of said ceiling openings, said weight extending through or into and essentially filling both of the co-axial openings in its bottom position.

9. An apparatus according to any of claims 5-8, **characterized** in that each of the releasable holding means is formed by a clamp plate (34) which is tiltable about a horizontal axis and is in contact with the rod-shaped lifting means (24) in its active state such as to allow upward, but to prevent downward movement of it, a solenoid (36), when energized, being adapted to bring the clamp plate into an inactive state in which it allows downward movement of the lifting means.

10. A container (11) for use in the apparatus according to any of claims 5-9 and consisting of a material of poor heat conduction, **characterized** in that the bottom and/or the cover (27) has a central opening which is closed by a disc of metal of good heat conduction.

**Patentansprüche**

1. Verfahren zur Heizung flüssiger Muster in betreffenden Behältern (11) auf eine vorbestimmte Referenztemperatur, mit Schritten zum

Heizen der flüssigen Muster mit Hochfrequenzenergie in einer Heizkammer (7), die gemeinsam für eine Vielzahl von flüssigen Mustern vorgesehen ist,

aufeinanderfolgenden Einführen der flüssigen Muster in die Heizkammer über eine Vielzahl von Muster-Handhabungsmitteln, wovon

jedes eines der Muster aufnimmt, jedes die Heizkammer (7) in bezug auf die Hochfrequenzenergie jedesmal dann abschließt, wenn ein flüssiges Muster in der Heizkammer aufgenommen worden ist,

jedes die Temperatur des betreffenden flüssigen Musters während des Heizens erfaßt und die Temperatur mit einer vorbestimmten Referenztemperatur vergleicht,

jedes sein betreffendes Muster individuell in Abhängigkeit von dem Vergleichsergebnis aus der Heizkammer entnimmt und jedes die Heizkammer (7) in bezug auf die Hochfrequenzenergie jedesmal dann abschließt, wenn das flüssige Muster aus der Heizkammer (7) entnommen worden ist,

während flüssige Muster, die in der Heizkammer (7) verbleiben, weiter geheizt werden.

2. Verfahren nach Anspruch 1, **gekennzeichnet** durch Anheben der Behälter (11) der Reihe nach in die Heizkammer (7) hinein von einem Transportmittel (28), das sich unterhalb und längs der Heizkammer erstreckt, und Freigeben jedes Behälters zum Rückführen zu dem Transportmittel mittels der Schwerkraft, wenn deren Inhalte die Referenztemperatur erreicht haben.

3. Verfahren nach Anspruch 2, dadurch **gekennzeichnet,** daß sich die Heizkammer (7) und das Transportmittel (28) als eine Einheit in einer ringförmigen Bahn bewegen.

4. Vorrichtung zur Heizung flüssiger Muster in betreffenden Behältern (11) auf eine vorbestimmte Referenztemperatur, mit einer Heizkammer (7), in der die flüssigen Muster mittels Hochfrequenzenergie geheizt werden,

wobei die Heizkammer (7) eine Vielzahl von Muster-Positionen für zu heizende Muster umfaßt,

dadurch gekennzeichnet, daß die Vorrichtung eine Vielzahl von Muster-Handhabungsmitteln zum aufeinanderfolgenden Einführen der Behälter (11) in die Heizkammer (7) umfaßt, wobei jedes individuelle Muster-Handhabungsmittel enthält:

Mittel zum Abschließen der Heizkammer (7) in bezug auf die Hochfrequenzenergie jedesmal dann, wenn ein Muster in der Heizkammer (7) aufgenommen worden ist,

einen Sensor (5) zum Erfassen der Temperatur jedes individuellen flüssigen Musters während des Heizens,

einen Mechanismus zum Entnehmen der betreffenden Behälter (11) aus der Heizkammer (7), wenn das flüssige Muster seine Referenztemperatur erreicht hat, und

Abschließmittel für Hochfrequenzenergie, die die Heizkammer abschließen, wobei die Abschließmittel wirksam mit deren betreffenden Entnahmemechanismus zum Abschließen der Heizkammer, wenn der Behälter (11) aus der Heizkammer (7) entnommen worden ist, verbunden ist,

so daß das Heizen der verbleibenden Muster fortgeführt werden kann.

5. Vorrichtung nach Anspruch 4, dadurch **gekennzeichnet,** daß sie ein ringförmiges Transportmittel (28) hat, das um eine vertikale Achse drehbar ist und mit in Abständen voneinander angeordneten vertikal bewegbaren Plattformen (29) ausgebildet ist, um die betreffenden Behälter (11) zu tragen, und daß die Heizkammer (7) fest mit dem Transportmittel verbunden ist und mit einem Abstand angeordnet ist, der größer als die Behälterhöhe oberhalb der Plattformen ist, wenn diese sich in ihren unteren Positionen befinden, daß Mittel (6, 24) vorgesehen sind, um jede Plattform (29) derart anzuheben, daß ein Behälter (11), der von ihr getragen wird, aufwärts in die Heizkammer (7) durch eine Öffnung in deren Boden bewegt wird, und daß entriegelbare Haltemittel (34) vorgesehen sind, um jeden Behälter (11) in der Heizkammer (7) zu halten, bis das enthaltene flüssige Muster die Referenztemperatur erreicht hat.

6. Vorrichtung nach Anspruch 5, dadurch **ge-**

**kennzeichnet,** daß ein Temperatur-Sensor (5) derart auf jeder Plattform (29) angeordnet ist, daß er einen Behälter (11), der auf der Plattform (29) plaziert ist, berührt.

7. Vorrichtung nach Anspruch 6, dadurch **gekennzeichnet,** daß jede Plattform (29) zusammengesetzt ist aus einem Träger-Ring und einem stangenförmigen, axial bewegbaren Hebemittel (24), dessen oberer Endteil sich aufwärts in den Ring hinein erstreckt und derart mit dem Ring verbunden ist, daß die Teile eine begrenzte axiale Beweglichkeit in Beziehung zueinander haben, wobei der Temperatur-Sensor (5) derart auf dem Ende des Hebemittels plaziert ist, daß er unterhalb bzw. oberhalb der Tragseite des Trägerrings vorhanden ist, wenn sich die Hebestange in ihrer unteren Position bzw. in ihrer oberen Position in bezug auf den Trägerring befindet, und daß eine stationäre Rampenbahn (6) vorgesehen ist, um die Aufwärtsbewegung des stangenförmigen Hebemittels zu veranlassen.

8. Vorrichtung nach Anspruch 5, 6 oder 7, dadurch **gekennzeichnet**, daß die Heizkammer (7) nach oben hin durch eine Deckenplatte definiert ist, die Öffnungen hat, welche koaxial mit den Öffnungen in dem Boden angeordnet sind, und daß ein Gewicht (9) axial in jeder der Decken-Öffnungen bewegbar ist, wobei sich das Gewicht durch oder in beide der koaxialen Öffnungen erstreckt und diese im wesentlichen in dessen unterer Position füllt.

9. Vorrichtung nach einem der Ansprüche 5 bis 8, dadurch **gekennzeichnet,** daß jedes der entriegelbaren Haltemittel durch eine Klemmplatte (34) gebildet ist, die um eine horizontale Achse kippkbar ist und derart in ihrem aktiven Zustand in Berührung mit dem stangenförmigen Hebemittel (24) steht, daß eine Aufwärtsbewegung gestattet ist, jedoch eine Abwärtsbewegung desselben verhindert wird, und daß ein Elektromagnet (36) vorgesehen ist, der, wenn er erregt ist, dazu bestimmt ist, die Klemmplatte in einen inaktiven Zustand zu bringen, in dem sie eine Abwärtsbewegung des Hebemittels gestattet.

10. Behälter (11) zur Benutzung in der Vorrichtung nach einem der Ansprüche 5 bis 9 und bestehend aus einem Material mit schlechter Wärmeleitung, dadurch **gekennzeichnet,** daß der Boden und/oder der Deckel (27) eine zentrale Öffnung hat, die durch eine Platte aus Metall mit guter Wärmeleitung verschlossen ist.

## Revendications

1. Procédé pour chauffer des échantillons de liquide dans des récipients respectifs (11) jusqu'à une température de référence prédéterminée, consistant à :

chauffer les échantillons de liquide avec une énergie haute fréquence dans une chambre chauffante (7) commune à une multiplicité d'échantillons de liquide ,

introduire successivement les échantillons de liquide dans la chambre chauffante par l'intermédiaire d'une multiplicité de moyens de manutention d'échantillons,

chacun de ces moyens recevant un des échantillons,

chacun de ces moyens obturant de façon hermétique la chambre chauffante (7) vis-à-vis de l'énergie haute fréquence chaque fois qu'un échantillon de liquide a été reçu dans la chambre chauffante,

chacun de ces moyens mesurant la température de l'échantillon de liquide respectif pendant le chauffage et comparant la température mesurée à une température de référence prédéterminée,

chacun retirant individuellement l'échantillon associé de la chambre chauffante en fonction de cette comparaison,

et chacun obturant de façon hermétique la chambre chauffante (7) vis-à-vis de l'énergie haute fréquence chaque fois que chaque échantillon de liquide a été retiré de la chambre chauffante (7),

tandis que les échantillons de liquide restant dans la chambre chauffante (7) continuent à être chauffés.

2. Procédé selon la revendication 1, **caractérisé** en ce que les récipients (11) sont successivement levés pour être amenés dans la chambre chauffante (7) depuis un moyen de transport (28) s'étendant en dessous et le long de la chambre chauffante, et en ce que chaque récipient est libéré pour retourner sur le moyen de transport par la gravité lorsque son contenu a atteint la température de référence.

3. Procédé selon la revendication 2, **caractérisé** en ce que la chambre chauffante (7) et les moyens de transport (28) se déplacent en tant qu'unité dans un trajet annulaire.

4. Appareil pour chauffer des échantillons de liquide dans des récipients respectifs (11) jusqu'à une température de référence prédéterminée, et comprenant une chambre chauffante (7) dans laquelle les échantillons de liquide sont chauffés par une énergie haute fréquence, cette chambre chauffante (7) comprenant une multiplicité de positions d'échantillons pour les échantillons à chauffer,

caractérisé en ce que l'appareil comprend une multiplicité de moyens de manutention d'échantillons pour introduire successivement les récipients (11) dans la chambre chauffante (7), chaque moyen de manutention d'échantillon individuel comprenant :

des moyens pour obturer de façon hermétique la chambre chauffante (7) vis-à-vis de l'énergie haute fréquence chaque fois qu'un échantillon a été reçu dans la chambre chauffante (7):

un capteur (5) pour mesurer la température de chaque échantillon de liquide individuel pendant le chauffage;

un mécanisme pour évacuer les récipients respectifs (11) de la chambre chauffante (7) lorsque l'échantillon de liquide a atteint sa température de référence;

et des moyens de fermeture hermétique vis-à-vis de l'énergie haute fréquence obturant de manière hermétique cette chambre chauffante, ces moyens d'obturation étant fonctionnellement reliés au mécanisme d'évacuation respectif pour obturer de façon hermétique la chambre chauffante lorsque le récipient (11) a été retiré de la chambre chauffante (7),

de telle sorte que le chauffage des échantillons restants peut continuer.

5. Appareil selon la revendication 4, **caractérisé** en ce qu'il a un dispositif de transport annulaire (28) tournant autour d'un axe vertical et comportant des plates-formes espacées verticalement mobiles (29) pour supporter les récipients respectifs (11) et en ce que la chambre chauffante (7) est solidarisée du dispositif de transport et est disposée à une distance plus grande que la hauteur du récipient au-dessus des plates-formes lorsque celles-ci sont dans leur position basse, des moyens (6,24) étant prévus pour lever chaque plate-forme (29) de façon qu'un récipient (11) supporté par la plate-forme est déplacé vers le haut dans la chambre chauffante (7) à travers une ouverture dans le fond de celle-ci, des moyens de tenue libérables (34) étant prévus pour retenir chaque récipient (11) dans la chambre chauffante (7) jusqu'à ce que l'échantillon de liquide contenu ait atteint la température de référence.

6. Appareil selon la revendication 5, **caractérisé** en ce qu'un capteur de température (11) est placé sur chaque plate-forme (29) de manière à être en contact avec un récipient (11) placé sur la plate-forme (29).

7. Appareil selon la revendication 6, **caractérisé** en ce que chaque plate-forme (29) est constituée par un anneau porteur et un élément de levée axialement mobile en forme de tige (24), dont la partie terminale supérieure s'étend vers le haut dans l'anneau porteur et est raccordée à celui-ci de telle sorte que les parties ont une mobilité axiale limitée l'une par rapport à l'autre, le capteur de température (5) étant placé sur l'extrémité du dispositif de levée de façon à se trouver plus bas que la face support de l'anneau porteur lorsque la tige de levée est dans sa position basse par rapport à l'anneau porteur, et à se trouver plus haut que la face support de l'anneau porteur lorsque la tige de levée est dans sa position haute par rapport à l'anneau porteur, une rampe fixe (6) étant prévue pour provoquer le mouvement vers le haut du dispositif de levée en forme de tige.

8. Appareil selon l'une des revendications 5, 6 ou 7, **caractérisé** en ce que la chambre chauffante (7) est délimitée vers le haut par une plaque formant plafond ayant des ouvertures coaxiales aux ouvertures dans le fond, et en ce qu'un poids (9) peut se déplacer axialement dans chacune de ces ouvertures du plafond, ce poids s'étendant à travers ou dans les deux ouvertures coaxiales et les remplissant pratiquement dans sa position basse.

9. Appareil selon l'une des revendications 5 à 8, **caractérisé** en ce que chacun des moyens de tenue libérables est constitué par une plaque de blocage (34) qui peut basculer autour d'un axe horizontal et qui est en contact avec le dispositif de levée en forme de tige (24) dans son état actif de façon à permettre au dispositif de levée de se déplacer vers le haut, tout en l'empêchant de se déplacer vers le bas, un solénoïde (36), lorsqu'il est excité, étant adapté pour amener la plaque de blocage en position de repos, position dans laquelle elle permet le mouvement vers le bas du dispositif de levée.

10. Récipient (11) destiné à être utilisé dans l'appareil selon l'une des revendications 5 à 9, et étant constitué en un matériau de mauvaise conductibilité thermique, **caractérisé** en ce que le fond et/ou le couvercle (27) a une ouverture centrale qui est fermée par un disque métallique ayant une bonne conductibilité thermique.

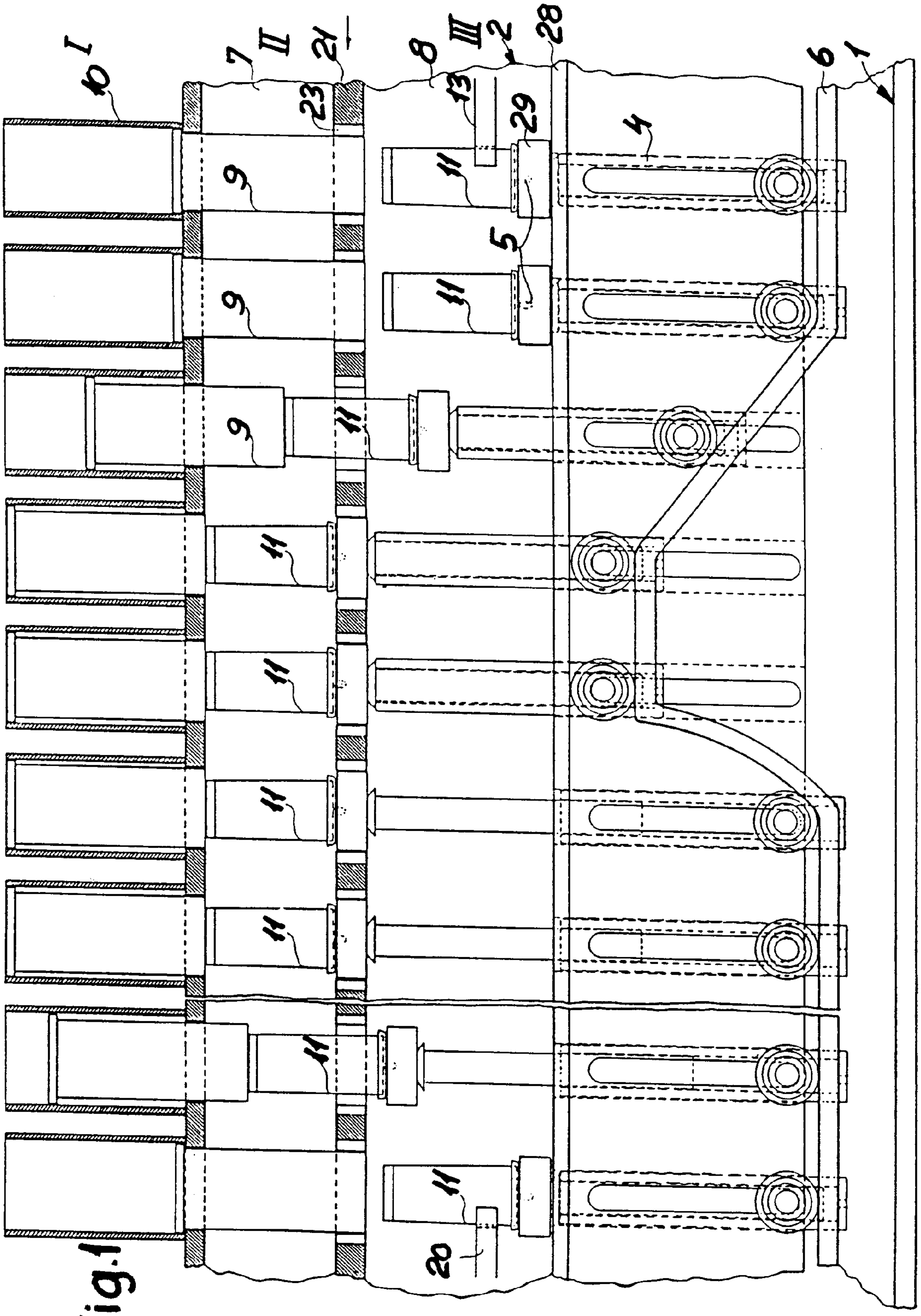


Fig.1

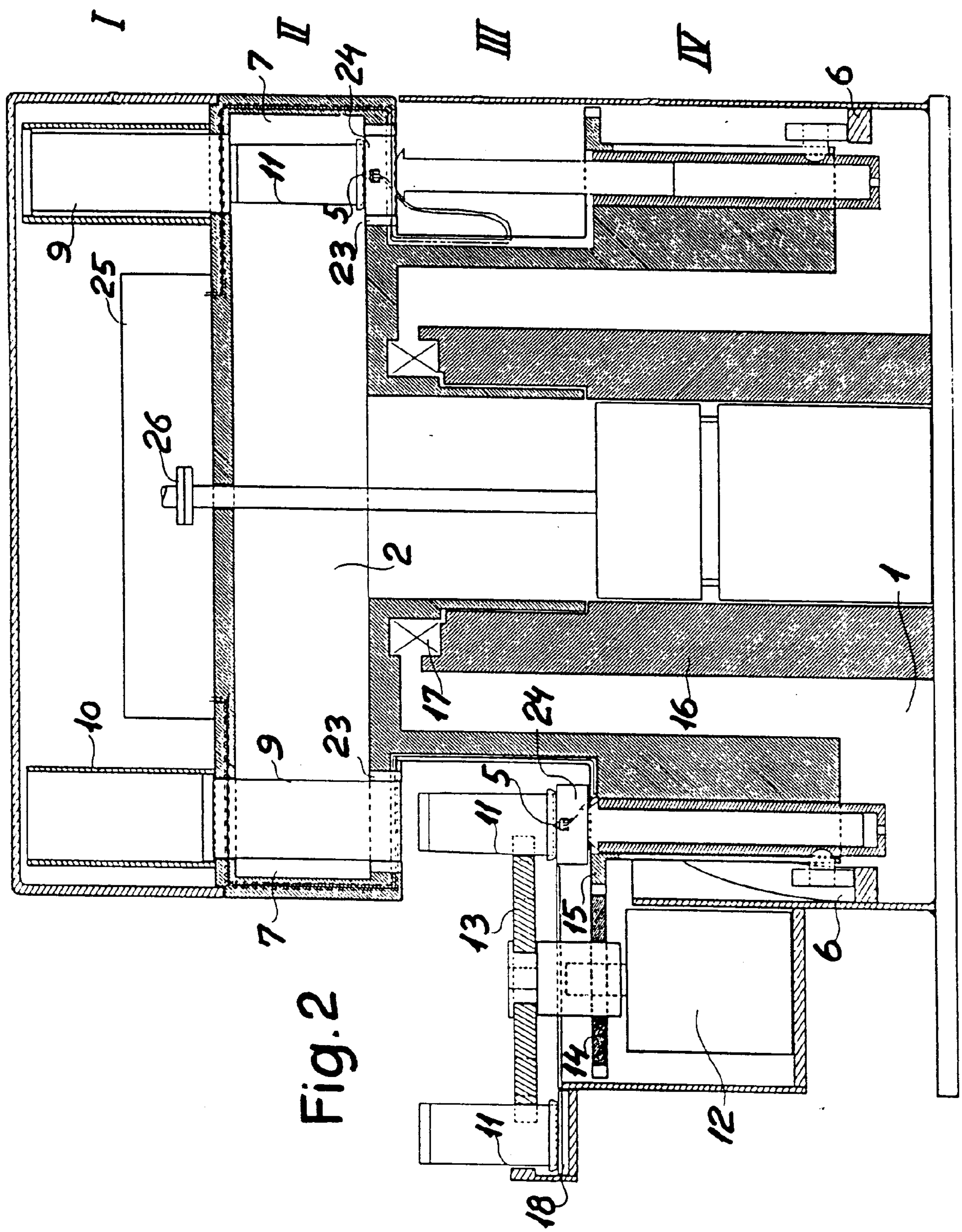


Fig.2

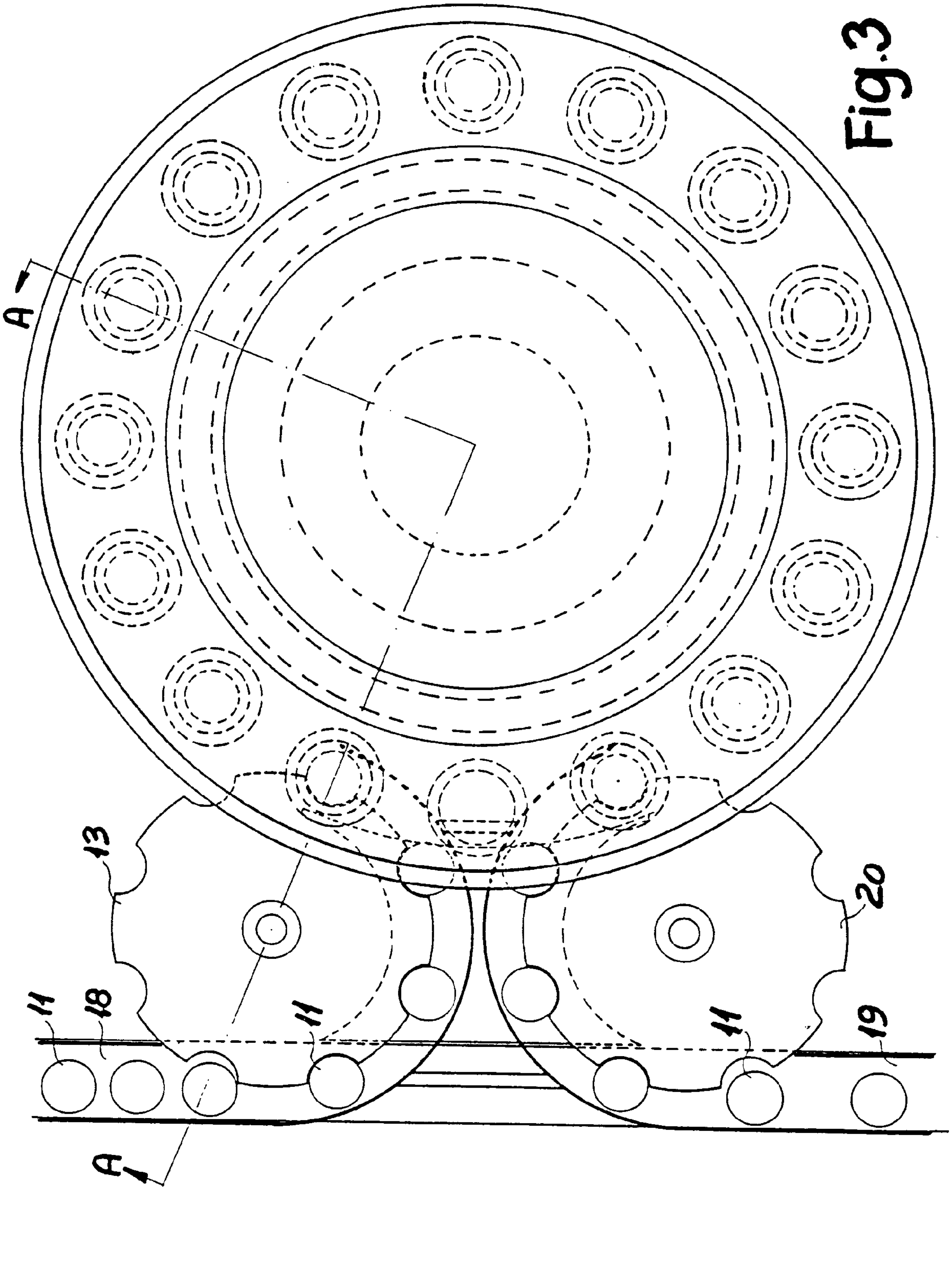


Fig.3

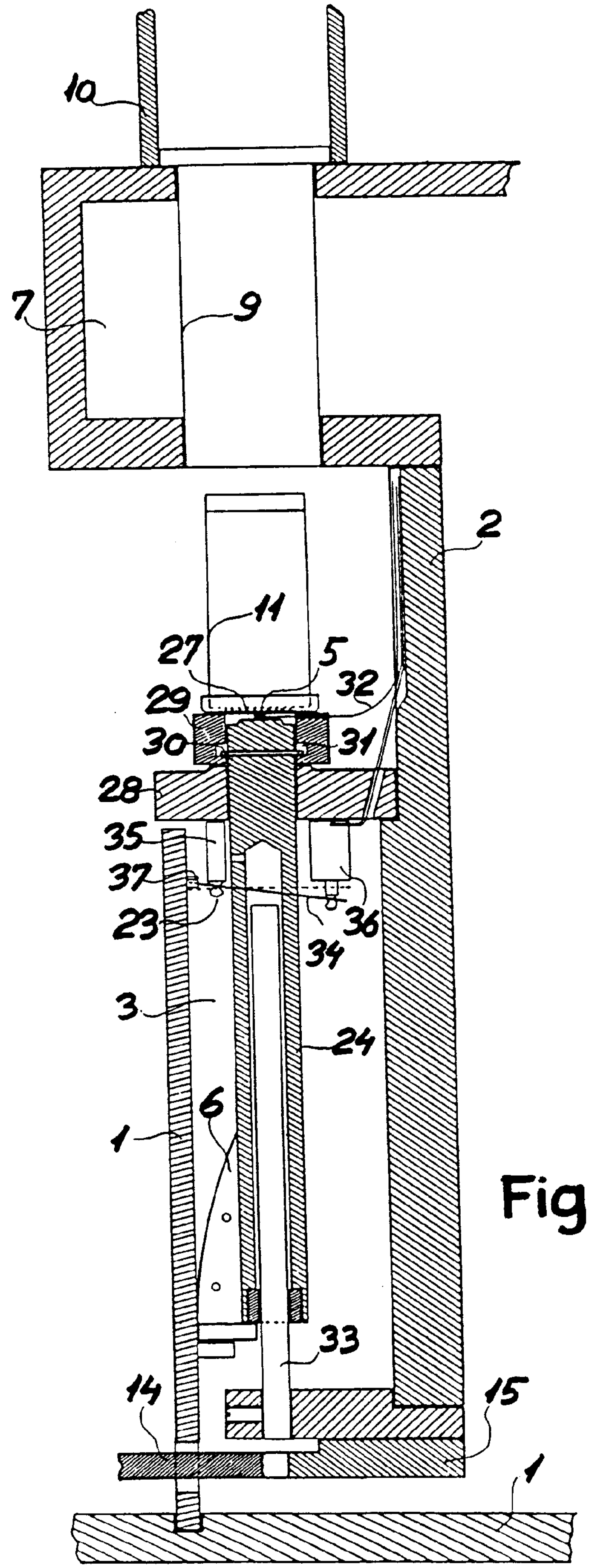


Fig. 4